# EUROPÄISCHE PATENTSCHRIFT

(11) **EP 4 037 642 B1**
(45) Veröffentlichungstag und Bekanntmachung des Hinweises auf die Patenterteilung: **06.11.2024**
(21) Anmeldenummer: 20767743.6
(22) Anmeldetag: 01.09.2020
(51) Int. Cl.: A61K 8/27, A61K 8/29, A61Q 17/04, A61K 8/20, A61K 8/49

(54) **NEUES, BISMUTHOXYCHLORID ENTHALTENDES SONNENSCHUTZMITTEL ZUM SCHUTZ VOR BLAUEM LICHT**
NOVEL SUN PROTECTIVE AGENT CONTAINING BISMUTH OXYCHLORIDE FOR PROTECTION AGAINST BLUE LIGHT
NOUVEL AGENT DE PROTECTION SOLAIRE CONTENANT DE L'OXYCHLORURE DE BISMUTH POUR LA PROTECTION CONTRE LA LUMIÈRE BLEUE

(30) Priorität: 01.10.2019 DE 102019215113
(43) Veröffentlichungstag der Anmeldung: 10.08.2022
(73) Patentinhaber: Beiersdorf AG, 22529 Hamburg (DE)
(72) Erfinder: HEITMANN, Birgit, 22885 Barsbüttel (DE); KAACK, Jorina, 24558 Henstedt-Ulzburg (DE); BLECKMANN, Andreas, 22926 Ahrensburg (DE)
(74) Vertreter: Beiersdorf AG
(86) Internationale Anmeldenummer: PCT/EP2020/074267
(87) Internationale Veröffentlichungsnummer: WO 2021/063610

(56) Entgegenhaltungen:
- EP-A1- 2 813 264
- EP-A1- 3 103 433
- CN-A- 109 077 952
- CN-A- 109 908 020
- KR-A- 20190 083 832
- DATABASE GNPD [online] MINTEL; 26 September 2019 (2019-09-26), ANONYMOUS: "Eraser Foam", XP055743181, retrieved from https://www.gnpd.com/sinatra/recordpage/6908779/ Database accession no. 6908779
- DATABASE GNPD [online] MINTEL; 18 September 2013 (2013-09-18), ANONYMOUS: "BB Cream SPF 15", XP055743183, retrieved from https://www.gnpd.com/sinatra/recordpage/2176612/ Database accession no. 2176612
- DATABASE GNPD [online] MINTEL; 13 August 2014 (2014-08-13), ANONYMOUS: "BB Cream All-in-1 Skin Perfector", XP055743205, retrieved from https://www.gnpd.com/sinatra/recordpage/2600009/ Database accession no. 2600009
- DATABASE GNPD [online] MINTEL; 10 March 2020 (2020-03-10), ANONYMOUS: "Glow Screen SPF 40 PA+++", XP055743179, retrieved from https://www.gnpd.com/sinatra/recordpage/7259125/ Database accession no. 7259125
- DATABASE GNPD [online] MINTEL; 3 April 2020 (2020-04-03), ANONYMOUS: "Color Balm SPF 50 PA++++", XP055743178, retrieved from https://www.gnpd.com/sinatra/recordpage/7435499/ Database accession no. 7435499
- DATABASE GNPD [online] MINTEL; 23 March 2017 (2017-03-23), ANONYMOUS: "DD Cream SPF 50+ PA+++", XP055743176, retrieved from https://www.gnpd.com/sinatra/recordpage/4696677/ Database accession no. 4696677
- DATABASE GNPD [online] MINTEL; 30 October 2014 (2014-10-30), ANONYMOUS: "BB Cream All-In-1 Blemish Balm", XP055743182, retrieved from https://www.gnpd.com/sinatra/recordpage/2753779/ Database accession no. 2753779
- DATABASE GNPD [online] MINTEL; 30 October 2014 (2014-10-30), ANONYMOUS: "BB Cream All-In-1 Blemish Balm", XP055743182, retrieved from www.gnpd.com Database accession no. 2753779
- DATABASE GNPD [online] MINTEL; 13 August 2014 (2014-08-13), ANONYMOUS: "BB Cream All-in-1 Skin Perfector", XP055743205, retrieved from www.gnpd.com Database accession no. 2600009
- DATABASE GNPD [online] MINTEL; 18 September 2013 (2013-09-18), ANONYMOUS: "BB Cream SPF 15", XP055743183, retrieved from www.gnpd.com Database accession no. 2176612
- DATABASE GNPD [online] MINTEL; 26 September 2019 (2019-09-26), ANONYMOUS: "Eraser Foam", XP055743181, retrieved from www.gnpd.com Database accession no. 6908779

## Beschreibung

Die vorliegende Erfindung betrifft eine kosmetische Zubereitung enthaltend
a) Bismuthoxychlorid-Partikel mit einem Brechungsindex von 2,15 sowie
b) Titandioxid und/oder Zinkoxid mit einer Primärpartikelgröße von 10 bis 200 nm, sowie die Verwendung einer Kombination aus

a) Bismuthoxychlorid-Partikel mit einem Brechungsindex von 2,15 und
b) Titandioxid und/oder Zinkoxid mit einer Primärpartikelgröße von 10 bis 200 nm. in kosmetischen Zubereitungen, zum Schutz vor Strahlung im Wellenlängenbereich von 401 bis 500 nm.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Sonnenschutzmittel enthalten üblicherweise Filtersubstanzen, die im UV-A und UV-B Wellenlängenbereich die elektromagnetische Strahlung absorbieren und/oder reflektieren. In jüngerer Zeit ist nun die so genannte "Blaulicht-Strahlung", d.h. elektromagnetische Strahlung im Wellenlängenbereich von 400 bis 500 nm in den Fokus der Forschung gerückt. Es wurde gefunden, dass dieses blaue Licht tiefer in die Haut eindringt als UV-A bzw. UV-B-Strahlung und dadurch eine Gefahr für alle Hautschichten darstellt. Blaues Licht erzeugt in der Haut oxidativen Stress sowohl bei der reaktiven Sauerstoffspezies (reactive oxygen species: ROS) als auch bei der reaktiven Stickstoffspezies (reactive nitrogen species: RNS). Es weicht die epidermale Barriere auf und schädigt die extrazelluläre Matrix. Hyperpigmentierung und die Zerstörung der Proteine und Lipide können die Folge sein. Alle diese Effekte können zu einer vorzeitigen Hautalterung führen und es ist aufgrund dieser Erkenntnisse auch davon auszugehen, dass die weiteren Inhaltsstoffe von Sonnenschutzmitteln (z.B. Lipide, Antioxidantien) selbst ebenfalls Schaden nehmen, wenn sie der Blaulicht-Strahlung ausgesetzt sind.

Es war daher die Aufgabe der vorliegenden Erfindung eine kosmetische Zubereitung (insbesondere ein Sonnenschutzmittel) zu entwickeln, dass vor den Schäden durch die Blaulicht-Strahlung schützt, wobei sich dieser Schutz sowohl das Sonnenschutzmittel an sich, als auch die damit behandelte Haut umfasst.

Überraschend gelöst wird die Aufgabe durch ein kosmetisches Sonnenschutzmittel gemäß Anspruch 1.

Überraschend gelöst wird die Aufgabe ferner durch eine Kombination gemäß Anspruch 2.

Zwar kennt der Stand der Technik die CN 109 908 020 A, EP 2 813 264 A1, EP 3 103 433 A1, CN 109 077 952 A, KR 2019 0083832 A sowie die Datenbank-Einträge in der GNPD-Datenbank von Mintel mit den Eintragungsnummern 2753779 (BB Cream All-in-1 Blemish), 2600009 (BB Cream All-in.1 Skin Perfector), 2176612 (BB Cream SPF 15), und6908779 (Eraser Foam), doch konnten diese Offenbarungen nicht den Weg zur vorliegenden Erfindung weisen.

Im Rahmen der vorliegenden Offenbarung beziehen sich die Formulierungen "erfindungsgemäß", "erfindungsgemäße Zubereitung" etc. immer auf die erfindungsgemäßen Zubereitungen und Verwendungen, d.h. auch auf Zubereitungen, in denen die erfindungsgemäße Verwendung verwirklicht wird.

Überraschend und für den Fachmann nicht vorhersehbar war die deutlich verbesserte Schutzleistung, wenn man Titandioxid bzw. Zinkoxid mit dem erfindungsgemäßen Bismuthoxychlorid kombiniert.

Dabei hat es sich insbesondere als erfindungsgemäß bevorzugt herausgestellt, wenn das Bismuthoxychlorid eine spezifische Oberfläche von 230-350m²/kg aufweist. Diese wird erfindungsgemäß aus der Teilchengröße (siehe unten) und der Dichte berechnet.

Das erfindungsgemäße Bismuthoxychlorid ist ferner dadurch als erfindungsgemäß bevorzugt gekennzeichnet, wenn seine Teilchengröße so gewählt wird, dass 10% Volumenanteile kleiner als 1-1,5 µm, 50% Volumenanteile kleiner als 3-6,5 µm und 90% Volumenanteile kleiner 8-15µm beträgt. Dabei wird die Teilchengrößenverteilung erfindungsgemäß nach dem Modell der Mie-Theorie berechnet.

Es ist erfindungsgemäß bevorzugt, wenn die Schüttdichte des Bismuthoxychlorides 7,70-7,75 g/cm³ und besonders bevorzugt 7,72 g/cm³ beträgt.

Alle Werte für das Bismuthoxychlorid werden im Trockenzustand bestimmt.

Das erfindungsgemäße Bismuthoxychlorid kann beispielsweise bei der Firma Merck unter dem Handelsnamen RonaFlair ESQ, Art. Nummer 117061 oder bei der Firma Textron unter dem Handelsnamen Evoshield käuflich erworben werden.

Es ist erfindungsgemäß von Vorteil, wenn die Zubereitung das Bismuthoxychlorid in einer Konzentration von 0,1 bis 10 Gewichts-%, bezogen auf das Gesamtgewicht der Zubereitung, in dieser enthalten ist. Dabei ist eine Konzentration von 1 bis 5,0 Gewichts-%, bezogen auf das Gesamtgewicht der Zubereitung, erfindungsgemäß bevorzugt.

Als erfindungsgemäß vorteilhaftes Titandioxid wird dabei Titandioxid in der Kristallstruktur Rutil mit einer Primärpartikelgröße von 2 -100 nm verwendet.

Es ist erfindungsgemäß bevorzugt, wenn die Primärpartikelgröße des erfindungsgemäßen Titandioxides im Bereich zwischen 5 und 50 nm liegt.

Erfindungsgemäß vorteilhafte Ausführungsformen der vorliegenden Erfindung sind dabei dadurch gekennzeichnet, dass das Titandioxid mit Silica (Siliziumdioxid und/oder Kieselsäure) beschichtet ist.

Es ist dabei erfindungsgemäß bevorzugt, wenn das mit Silica beschichtete Titandioxid auf der äußeren Seite der Silica-Schicht (d.h. auf der dem Titandioxid-abgewandten Seite) eine Schicht aus Dimethicone, Simethicone oder Methicone aufweist. Unter diesen Stoffen ist Dimethicone dabei erfindungsgemäß besonders bevorzugt.

Es ist erfindungsgemäß vorteilhaft, wenn die sekundäre Partikelgröße des Titandioxid in der Kristallstruktur Rutil mit einer Primärpartikelgröße von 2 -100 nm zwischen 0,05 und 50 µm beträgt. Dabei ist der Bereich von 0,1 bis 1 µm erfindungsgemäß bevorzugt.

Die primäre und sekundäre Partikelgröße entnimmt der Fachmann folgender Literaturstelle: SCCS/1516/13 Opinion on Titanium Dioxide (nano form) Colipa No S75 der Cosmetics Europe personal care association. Entsprechende Veröffentlichungen gibt es auch zur Primärpartikelgröße von Zinkoxid (SCCS/1489/12 Opinion).

Als Zinkoxid werden bevorzugt Partikel eingesetzt, deren Primärpartikelgröße kleiner 150 nm beträgt. Es ist erfindungsgemäß vorteilhaft mit Triethoxycaprylylsilane beschichtet.

Es ist erfindungsgemäß von Vorteil, wenn das Titandioxid und/oder Zinkoxid in einer Gesamtmenge von 1 bis 30 Gewichts-%, bezogen auf das Gesamtgewicht der Zubereitung, in dieser enthalten ist.

Es gibt dabei drei erfindungsgemäße Ausführungsformen:
i) Zubereitungen enthaltend Bismuthoxychlorid sowie
b) Titandioxid.

In diesem Falle betragen die erfindungsgemäß vorteilhaften Einsatzkonzentrationen für das Bismuthoxychlorid von 0,1 bis 10 Gewichts-% und für Titandioxid von 0,5 bis 25 Gewichts-%, jeweils bezogen auf das Gesamtgewicht der Zubereitung.

ii) Zubereitungen enthaltend Bismuthoxychlorid sowie
b) Zinkoxid.

In diesem Falle betragen die erfindungsgemäß vorteilhaften Einsatzkonzentrationen für das Bismuthoxychlorid von 0,1 bis 10 Gewichts-% und für Zinkoxid von 0,5 bis 25 Gewichts-%, jeweils bezogen auf das Gesamtgewicht der Zubereitung.

iii) Zubereitungen enthaltend Bismuthoxychlorid sowie
b) Titandioxid und Zinkoxid.

In diesem Falle betragen die erfindungsgemäß vorteilhaften Einsatzkonzentrationen für das Bismuthoxychlorid von 0,1 bis 10 Gewichts-%, für Titandioxid von 0,5 bis 10 Gewichts-%, und für Zinkoxid von 0,5 bis 20 Gewichts-%, jeweils bezogen auf das Gesamtgewicht der Zubereitung.

Vorteilhafte Ausführungsformen der vorliegenden Erfindung sind dadurch gekennzeichnet, dass die Zubereitung zusätzlich Interferenz-Pigmente enthält. Diese können erfindungsgemäß vorteilhaft in einer Konzentration von 0,5 bis 6 Gewichts-%, bezogen auf das Gesamtgewicht der Zubereitung, in dieser eingesetzt werden.

Interferenzpigmente sind Effektpigmente, deren Wirkung vorwiegend oder ganz auf Interferenz von Licht an dünnen, hochbrechenden Schichten beruht. Sie zeichnen sich dadurch aus, dass sie einen winkelabhängigen Farbeindruck erzeugen, der als Farbtonflop bezeichnet wird.

Als erfindungsgemäß bevorzugte Interferenz-Pigmente können eingesetzt werden: Titanium Dioxide (and) Mica (and) Tin Oxide; Mica (and) Titanium Dioxide (and) Silica; Calcium Sodium Borosilicate (and) Silica (and) Titanium Dioxide CI 77891; Cl 77891 (Titanium Dioxide) (and) Mica (and) Tin Oxide; Mica (and) Titanium Dioxide (and) Silica (and) Tin Oxide; Calcium Sodium Borosilicate (and) Titanium Dioxide (and) Tin Oxide; Calcium Aluminum Borosilicate (and) Titandium Dioxide; Mica (and) Titanium Dioxide

Darüber hinaus ist es erfindungsgemäß von Vorteil, wenn die Zubereitung 2,2'-Methylen-bis-(6-(2H-benzotriazol-2-yl)-4-(1,1,3,3-tetramethylbutyl)-phenol) (INCI: Methylene Bis-Benzotriazolyl Tetramethylbutylphenol) und/oder 2,4,6-Tris-(biphenyl)-1,3,5-triazin (INCI: Tris-Biphenyl Triazine) enthält.

Es ist erfindungsgemäß vorteilhaft, wenn die Zubereitung einen oder mehrere UV-Filter enthält, die gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)-benzolsulfonsäuresalze; 2-Methyl-5-(2-oxo-3-bornylidenmethyl)sulfonsäuresalze; 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-Benzylidencampher; Ethylhexylsalicylat; Terephthalidendicampher-sulfonsäure und/oder deren Salze; 4-(Dimethylamino)-benzoesäure(2-ethylhexyl)ester; 2-Ethylhexyl-2-cyano-3,3-diphenylacrylat (Octocrylen), 4-(Dimethylamino)benzoesäure-amylester; 4-Methoxybenzalmalon-säuredi(2-ethylhexyl)ester; 2-Hydroxy-4-methoxy-4'-methylbenzophenon; 2,2'-Dihydroxy-4-methoxybenzophenon; Homomenthylsalicylat (Homosalate); 2-Ethylhexyl-2-hydroxybenzoat; Dimethicodiethylbenzalmalonat; 3-(4-(2,2-bis Ethoxycarbonylvinyl)-phe-noxy)propenyl)-methoxysiloxan / Dimethylsiloxan - Copolymer; 4-(tert.-Butyl)-4'-methoxydiben-zoylmethan; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); Merocyanine.

Erfindungsgemäß bevorzugt ist dabei der Einsatz von 2,4,6-Tris-[anilino-(p-carbo-2'-ethyl-1'-hexyloxy)]-1,3,5-triazin (INCI: Ethylhexyl Triazone), 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine), 2-Ethylhexyl 2-hydroxybenzoat (INCI: Ethylhexyl Salicylate) und 2-Phenylbenzimidazol-5-sulfonsäuresalzen.

Erfindungsgemäß vorteilhafte Ausführungsformen der vorliegenden Erfindung sind dadurch gekennzeichnet, dass die Zubereitung in Form einer alkoholischen Lösung oder einer Emulsion vorliegt.

Es ist erfindungsgemäß bevorzugt ist es dabei, wenn die Zubereitung in Form einer Emulsion und besonders bevorzugt in Form einer O/W-Emulsion vorliegt.

Liegt die erfindungsgemäße Zubereitung in Form einer O/W-Emulsion vor, so ist sie erfindungsgemäß vorteilhaft dadurch gekennzeichnet, dass die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Natriumcetearylsulfat, Kaliumcetylphosphat, Polyglyceryl-10 Stearate , Natriumstearoylglutamat, enthält.

Diese erfindungsgemäßen O/W-Emulgatoren können erfindungsgemäß vorteilhaft in einer Konzentration von 0,001 bis 10 Gewichts-% und bevorzugt in einer Konzentration von 0,1 bis 7 Gewichts-%, bezogen auf das Gesamtgewicht der Zubereitung in dieser enthalten sein.

Es ist erfindungsgemäß bevorzugt, wenn die Zubereitung Natriumstearoylglutamat als Emulgator enthält.

Es ist ferner erfindungsgemäß vorteilhaft, wenn die Zubereitung Cetylalkohol, Stearylalkohol und/oder Glycerylstearat enthält.

Es ist erfindungsgemäß für die Kombination von Vorteil, wenn die erfindungsgemäße Zubereitung frei ist von Polyethylenglycol, Polyethylenglycolethern und Polyethylenglycolestern (sogenannten PEG-Derivaten).

Erfindungsgemäß vorteilhafte Ausführungsformen der Kombination sind ferner dadurch gekennzeichnet, dass die Zubereitung kein 3-(4-Methylbenzyliden)-campher, 2-Hydroxy-4-methoxybenzophenon (INCI: Oxybenzon), 4-Methoxyzimtsäure(2-ethylhexyl)ester, 4-Methoxyzimtsäureisoamylester und Ethylhexyl-2-cyano-3,3-diphenylacrylat (INCI: Octocrylen), keine Parabene, Methylisothiazolinon, Chlorphenesin, Chlormethylisothiazolinon und DMDM-Hydantoin enthält.

Es ist darüber hinaus vorteilhaft im Sinne der vorliegenden Erfindung, wenn die Zubereitung sphärische, hohle Silica-Partikel mit einem durchschnittlichen Teilchendurchmesser von 15 bis 20 µm, gemessen mittels Rasterelektronenmikroskopie, enthält.

Es ist ferner erfindungsgemäß von besonderem Vorteil, wenn die erfindungsgemäße Zubereitung Hexyl 2-[4-(diethylamino)-2-hydroxybenzoyl]benzoate (INCI: Diethylamino hydroxybenzoyl hexyl benzoate) und/oder 4-(tert.-Butyl)-4'-methoxydibenzoylmethan enthält.

Vorteilhafte Ausführungsformen der vorliegenden Erfindung sind ferner dadurch gekennzeichnet, dass die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol, Ethylhexylglycerin und/oder 4-Hydroxyacetophenon enthält.

Außerdem ist es erfindungsgemäß vorteilhaft, wenn die Zubereitung Tapiokastärke und/oder Silica Dimethyl Silylate enthält.

Nicht zuletzt sind die erfindungsgemäß vorteilhaften Ausführungsformen dadurch gekennzeichnet, dass die Zubereitung ein oder mehrere Verbindungen enthält, gewählt aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Ethylenbrassylat, Evernia Furfuracea Extract,

Evernia Prunastri Extract, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

Die erfindungsgemäße Zubereitung kann darüber hinaus wie ein übliches kosmetisches Sonnenschutzmittel zusammengesetzt sein. Dabei hat es sich als vorteilhaft herausgestellt, wenn die Zubereitung ein oder mehrere Feuchthaltemittel enthält.

Die erfindungsgemäße Zubereitung kann vorteilhaft Feuchthaltemittel enthalten. Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1 , Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel^{®}1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Erfindungsgemäß vorteilhafte Ausführungsformen der vorliegenden Erfindung sind dadurch gekennzeichnet, dass die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropylsebacate, Dicaprylylcarbonat, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, Isopropyl Palmitate, enthält.

Dabei ist es erfindugnsgemäß bevorzugt, wenn die Zubereitung Dibutyladipat, Butylene Glycol Dicaprylate/Dicaprate und/oder C12-C15 Alkylbenzoat enthält.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, etc..

Erfindungsgemäß vorteilhafte Ausführungsformen der vorliegenden Erfindung sind dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A enthält.

### Vergleichsversuch

Es wurden die folgenden Rezepturen hergestellt und der Schutz vor blauem Licht mit Hilfe der folgenden Methode bestimmt.

Messung des Schutzes vor blauem Licht nach der ISO 24443.

Jedes Produkt wurde auf 3 WW5-PMMA-Platten (1,3 mg / cm² +/- 1,5%) mit den folgenden Anwendungseigenschaften aufgetragen: zweiphasig, Phase A: Leichter Druck und kreisförmige Bewegung, Phase B: Druck höher als 150 g und Striche auf vier verschiedene Richtungen, Finger mit einem Fingerling bedeckt.

Nach der Gleichgewichtseinstellung nach 30 min bei 40 ° C wurde die Durchlässigkeit an 3 Punkten pro Platte (280 - 600 nm in Schritten von 1 nm) gemessen. Eine mit Glycerin aufgebrachte Platte diente als 100% Referenz.

Verwendetes Material: Plexiglass ^{®} WW5 (Schönberg GmbH & Co. KG, Germany) Messgerät: UV/VIS Spectrometer Lambda 650 S (PerkinElmer) ausgerüstet mit einer 150 mm integrierten Kugel.

Als Bismuthoxychlorid wurde RonaFlair ESQ, Art. Nummer 117061 der Firma Merck bzw. Evoshield der Firma Textron eingesetzt.

| inci | _hevl_96 | _hevl_161 | _hevl_165 | _hevl_171 |
|---|---|---|---|---|
| Bismuth Oxychloride (Ronaflair ESQ) | 0 | 5 | 2.5 | 2.5 |
| Ethylhexylglycerin | 0.25 | 0.25 | 0.25 | 0.25 |
| Dibutyl Adipate | 3 | 3 | 3 | 3 |
| C12-15 Alkyl Benzoate | 5 | 5 | 5 | 5 |
| Butylene Glycol Dicaprylate/Dicaprate | 4 | 4 | 4 | 4 |
| Glyceryl Stearate | 1 | 1 | 1 | 1 |
| Sodium Stearoyl Glutamate | 0.3 | 0.3 | 0.3 | 0.3 |
| Parfum | 0.3 | 0.3 | 0.3 | 0 |
| Parfum | 0 | 0 | 0 | 0.5 |
| Glycerin | 8.6 | 8.6 | 8.6 | 8.6 |
| Aqua + Sodium Hydroxide | 0.33 | 0.33 | 0.33 | 0.33 |
| Phenoxyethanol | 0.5 | 0.5 | 0.5 | 0.5 |
| Stearyl Alcohol | 1 | 1 | 1 | 1 |
| Acrylates/C10-30 Alkyl Acrylate Crosspolymer | 0.1 | 0.1 | 0.1 | 0.1 |
| Xanthan Gum | 0.4 | 0.4 | 0.4 | 0.4 |
| Aqua | 56.47 | 51.47 | 48.97 | 43.77 |
| Alcohol Denat. + Aqua | 6 | 6 | 6 | 6 |
| Aqua + Trisodium EDTA | 1 | 1 | 1 | 1 |
| Ethylhexyl Salicylate | 4 | 4 | 4 | 4 |
| Bis-Ethylhexyloxyphenol Methoxyphenyl Triazine | 1.75 | 1.75 | 1.75 | 1.75 |
| Ethylhexyl Triazone | 1.5 | 1.5 | 1.5 | 1.5 |
| Butyl Methoxydibenzoylmethane | 3.5 | 3.5 | 3.5 | 3.5 |
| Phenylbenzimidazole Sulfonic Acid | 1 | 1 | 1 | 1 |
| Titanium Dioxide (nano) + Silica + Dimethicone | 0 | 0 | 5 | 0 |
| Methylene Bis-Benzotriazolyl Tetramethylbutylphenol | 0 | 0 | 0 | 10 |
| Transmission in % [400-500 nm] | 97,79 % | 82,04 % | 69,72 % | 71,82 % |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Beispiele** | **1** | **2** | **3** | **4** | **5** | **6** | **7** | **8** | **9** | **10** | **11** | **12** |
|---|---|---|---|---|---|---|---|---|---|---|---|---|
| **INCI** | **m[% ]** | **m[% ]** | **m[% ]** | **m[% ]** | **m[% ]** | **m[% ]** | **m[% ]** | **m[% ]** | **m[%]** | **m[% ]** | **m[% ]** | **m[% ]** |
| Ronaflair ESQ | 2,5 | 5 | 5 | | | 2 | 6 | | | 3 | | |
| Evoshield | | | | 2,5 | 5 | | | 2 | 6 | | 3 | 3 |
| VP/Hexadecene Copolymer | 0,5 | 1 | 1 | | 0,5 | | 1 | | 0,5 | | | |
| Tocopherylacetate | 0,06 | 0,1 | 0,1 | 0,01 | 0,15 | 0,06 | 0,5 | 0,1 | 0,06 | 0,15 | 0,1 | 0,1 |
| Panthenol | 1 | 1,4 | 1,4 | | 0,5 | 0,5 | | 1 | 1 | | 0,5 | 0,5 |
| Ethylhexylglycerin | 0,3 | 0,5 | 0,5 | 0,25 | | | | 0,3 | | | 0,25 | 0,25 |
| 1,2-Hexandiole | | | | | 0,4 | | 1 | | | 1 | | |
| Methylpropanediole | | | | | | 0,2 | | | 0,2 | | | |
| C12-15 Alkylbenzoate | 4,5 | 6 | 6 | 3 | | | 6 | 7 | | 7,5 | | |
| Caprylic/Capric Triglycerides | | 4 | 4 | | | 2 | | | | 3 | | |
| Isopropyl Palmitate | | | | 4 | | | | | 3 | | 3 | 3 |
| Dimethicone | | | | | 2 | 1 | | | 0,5 | | | |
| Octyldodecanol | | | | | 2 | | | | | 2 | | |
| Ethylhexyl Cocoate | | | | | | | 1 | | | 2 | 3 | 3 |
| Myristyl Myristate | 2 | 1 | 1 | 1,5 | | | 1 | | | | | |
| Cetearyl Alcohol | | | | | 1 | | | 1,5 | | | 1 | 1 |
| Hydrogenated Coco-Glycerides | | | | | | 5 | | | 2 | | 1 | 1 |
| Butylene Glycol Dicaprylate/Dicaprate | 1 | | | 3 | 1,5 | | | 2 | | | | |
| Dicaprylyl Carbonate | 1 | | | | 3 | | 3 | | | 1,5 | | |
| C18-38 Alkyl Hydroxystearoyl Stearate | | | | | | 0,5 | | | | | | |
| Polyglyceryl-3 Methylglucose Distearate | | | | | 0,15 | | | | | 0,15 | | |
| Glyceryl Stearate Citrate | 2 | 3 | 3 | | | | 2 | | 2 | | | |
| Glyceryl Stearate | | | | 1 | | 1 | | | | | | |
| Sodium Stearoyl Glutamate | | | | | | | | 0,5 | | | | |
| Glyceryl Stearate SE | | | | | | | | 1 | | | 1 | 1 |
| Sodium Cetearal Sulfate | | | | 0,15 | | 0,15 | | | | | 0,15 | 0,15 |
| Silica dimethyl Silylate | | 0,5 | 0,5 | 0,5 | | 0,3 | 1 | 0,5 | | 1 | | |
| Tapioca starch | | | | | | | | 3 | 3 | | | |
| Parfum | 0,4 | 0,3 | 0,3 | 0,2 | 0,4 | | 0,4 | 0,5 | | 0,3 | 0,2 | 0,2 |
| Glycerin | 6 | 8 | 8 | 5 | 1 | 1 | 5 | 8 | 6 | 5 | 1 | 1 |
| Citric Acid | 0,07 | 0,09 | 0,09 | | | | 0,01 | | 0,00 8 | | | |
| Sodium Citrate | 0,16 | 0,15 | 0,15 | | | | 0,16 | | 0,17 | | | |
| Sodium Hydroxide | 0,05 | 0,08 | 0,08 | 0,07 | 0,08 | 0,05 | 0,03 | | 0,07 | 0,06 | 0,03 | 0,03 |
| Phenoxyethanol | 0,3 | 0,5 | 0,5 | 0,3 | 0,4 | 0,5 | 0,2 | | 0,5 | 0,3 | 0,4 | 0,4 |
| Methylparaben | 0,2 | 0,3 | 0,3 | 0,2 | 0,2 | 0,3 | | | 0,4 | 0,2 | 0,3 | 0,3 |
| Ethylparaben | 0,2 | 0,3 | 0,3 | 0,2 | 0,3 | | | | 0,2 | 0,3 | | |
| Stearyl Alcohol | 0,8 | 0,8 | 0,8 | | | 1 | | 0,8 | 1,5 | | | |
| Interference Pigment | 5 | | | | | 5 | | | | 5 | | |
| Silica | | | | 2 | | 5 | | | | | | 5 |
| Acrylates/C10-30Alkyl Acrylates Crosspolymer | 0,1 | 0,15 | 0,15 | 0,05 | | 0,2 | 0,1 | | 0,05 | 0,15 | 0,2 | 0,2 |
| Carbomer | | | | | | 0,15 | | | | | | |
| Hydroxyethylcellulose | | | | | | 0,1 | | | | | | |
| Xanthan Gum | 0,3 | 0,2 | 0,2 | 0,5 | 0,4 | | 0,3 | 0,4 | 0,2 | 0,3 | 0,3 | 0,3 |
| Cetyl Alcohol | 0,5 | 1 | 1 | | | | | | | 2 | | |
| Alcohol Denat | 4 | 6 | 6 | 4 | 8 | 10 | 4 | 6 | 6 | 8 | 10 | 10 |
| Trisodium EDTA | 1 | 0,5 | 0,5 | 1 | 1 | 1 | 0,5 | 0,5 | 1 | 1 | 1 | 1 |
| Homosalate | 9 | | | 9 | 9 | 9,5 | 9 | 9 | 9 | 9 | 9 | 9 |
| Octocrylene | 9 | 9 | 9 | | | 9,5 | | | 8 | 8 | | |
| Ethylhexyl Salicylate | 4,5 | | | 4,75 | 4,75 | 4,75 | 4,75 | 4,75 | 4,75 | 4,75 | 4,75 | 4,75 |
| Butyl Methoxydibebzoylmethan e | 4,5 | 4,5 | 4,5 | | 4,75 | 4,75 | 4,75 | 4,5 | 4,5 | 4,75 | 4,75 | 4,75 |
| Methylene Bis-Benzotriazolyl Tetramethylbutylphenol | 3 | | | | | | | | | | | |
| Titanium dioxide | 3 | 6 | | 1 | 2 | 1 | 1 | 1 | | 3,5 | 2 | |
| Zinc Oxide | | | 6 | 1 | 1 | 2 | 1 | | 1 | | | 5 |
| Bis-Ethylhexyloxyphenol Methoxyphenyl Triazine | | 3 | 3 | 4 | 4 | 3,5 | 4 | 4 | 3,5 | 3 | 4 | 4 |
| Ethylhexyl Triazone | | | | 3 | 3 | | 3 | 3 | | | 3 | |
| Diethylamino Hydroxybenzoyl Hexylbenzoate | | | | 8 | | | | | | | | |
| Polysilicone-15 | | 1 | 1 | | | | | | | | | |
| Phenylbenzimidazole Sulfonic Acid | | | | 1 | 1 | 1 | 1 | | 1,5 | 1,5 | 1 | 0,5 |
| Aqua | ad 100 | ad 100 | ad 100 | ad 100 | ad 100 | ad 100 | ad 100 | ad 100 | ad 100 | ad 100 | ad 100 | ad 100 |

Als Bismuthoxychlorid wurde RonaFlair ESQ, Art. Nummer 117061 der Firma Merck bzw. Evoshield der Firma Textron eingesetzt.

## Patentansprüche

1. Kosmetisches Sonnenschutzmittel enthaltend
a) Bismuthoxychlorid-Partikel mit einem Brechungsindex von 2,15 sowie
b) Titandioxid und/oder Zinkoxid mit einer Primärpartikelgröße von 10 bis 200 nm, **dadurch gekennzeichnet, dass** die Zubereitung frei ist von Polyethylenglycol, Polyethylenglycolethern und Polyethylenglycolestern (sogenannten PEG-Derivaten) und kein 3-(4-Methylbenzyliden)-campher, 2-Hydroxy-4-methoxybenzophenon (INCI: Oxybenzon), 4-Methoxyzimtsäure(2-ethylhexyl)ester, 4-Methoxyzimtsäureisoamylester und Ethylhexyl-2-cyano-3,3-diphenylacrylat (INCI: Octocrylen), keine Parabene, Methylisothiazolinon, Chlorphenesin, Chlormethylisothiazolinon und DMDM-Hydantoin enthält.

2. Kombination aus
a) Bismuthoxychlorid-Partikeln mit einem Brechungsindex von 2,15 und
b) Titandioxid und/oder Zinkoxid mit einer Primärpartikelgröße von 10 bis 200 nm in kosmetischen Zubereitungen zur Verwendung zum Schutz vor Strahlung im Wellenlängenbereich von 401 bis 500 nm.

3. Kosmetische Zubereitung nach Anspruch 1 oder Kombination nach Anspruch 2, **dadurch gekennzeichnet, dass** das Bismuthoxychlorid eine spezifische Oberfläche von 230-350m²/kg aufweist.

4. Kosmetische Zubereitung oder Kombination nach einem der vorhergehenden Ansprüche, **dadurch gekennzeichnet, dass** die Schüttdichte des Bismuthoxychlorides 7,70-7,75 g/cm³ beträgt.

5. Kosmetische Zubereitung oder Kombination nach einem der vorhergehenden Ansprüche, **dadurch gekennzeichnet, dass** die Zubereitung das Bismuthoxychlorid in einer Konzentration von 0,1 bis 10 Gewichts-%, bezogen auf das Gesamtgewicht der Zubereitung, in dieser enthalten ist.

6. Kosmetische Zubereitung oder Kombination nach einem der vorhergehenden Ansprüche, **dadurch gekennzeichnet, dass** Titandioxid und/oder Zinkoxid.in einer Gesamtmenge von 1 bis 30 Gewichts-%, bezogen auf das Gesamtgewicht der Zubereitung, in dieser enthalten ist.

7. Kosmetische Zubereitung oder Kombination nach einem der vorhergehenden Ansprüche, **dadurch gekennzeichnet, dass** die Zubereitung zusätzlich Interferenz-Pigmente enthält.

8. Kosmetische Zubereitung oder Kombination nach einem der vorhergehenden Ansprüche, **dadurch gekennzeichnet, dass** die Zubereitung 2,2'-Methylen-bis-(6-(2H-benzotriazol-2-yl)-4-(1,1,3,3-tetramethylbutyl)-phenol) (INCI: Methylene Bis-Benzotriazolyl Tetramethylbutylphenol) und/oder 2,4,6-Tris-(biphenyl)-1,3,5-triazin (INCI: Tris-Biphenyl Triazine) enthält.

9. Kosmetische Zubereitung oder Kombination nach einem der vorhergehenden Ansprüche, **dadurch gekennzeichnet, dass** die Zubereitung einen oder mehrere UV-Filter enthält, die gewählt werden aus der Gruppe der Verbindungen 2-Phenylbenzimidazol-5-sulfonsäure und/oder deren Salze; Phenylen-1,4-bis-(2-benz-imidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-Benzylidencampher; Ethylhexylsalicylat; Terephthalidendicampher-sulfonsäure und/oder deren Salze; 4-(Dimethylamino)-benzoesäure(2-ethylhexyl)-ester; 2-Ethylhexyl-2-cyano-3,3-diphenylacrylat (Octocrylen), 4-(Dimethylamino)benzoesäure-amylester; 4-Methoxybenzalmalon-säuredi(2-ethylhexyl)ester; 2-Hydroxy-4-methoxy-4'-methylbenzophenon; 2,2'-Dihydroxy-4-methoxybenzophenon; Homomenthylsalicylat (Homosalate); 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 4-(tert.-Butyl)-4'-methoxydibenzoylmethan; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); Merocyanine.

10. Kosmetische Zubereitung oder Kombination nach einem der vorhergehenden Ansprüche, **dadurch gekennzeichnet, dass** die Zubereitung in Form einer alkoholischen Lösung oder einer Emulsion vorliegt.

11. Kombination nach einem der vorhergehenden Ansprüche, **dadurch gekennzeichnet, dass** die Zubereitung frei ist von Polyethylenglycol, Polyethylenglycolethern und Polyethylenglycolestern (sogenannten PEG-Derivaten).

12. Kombination nach einem der vorhergehenden Ansprüche, **dadurch gekennzeichnet, dass** die Zubereitung kein 3-(4-Methylbenzyliden)-campher, 2-Hydroxy-4-methoxybenzophenon (INCI: Oxybenzon), 4-Methoxyzimtsäure(2-ethylhexyl)ester, 4-Methoxyzimtsäureisoamylester und Ethylhexyl-2-cyano-3,3-diphenylacrylat (INCI: Octocrylen), keine Parabene, Methylisothiazolinon, Chlorphenesin, Chlormethylisothiazolinon und DMDM-Hydantoin enthält.

13. Kosmetische Zubereitung oder Kombination nach einem der vorhergehenden Ansprüche, **dadurch gekennzeichnet, dass** die Zubereitung sphärische, hohle Silica-Partikel mit einem durchschnittlichen Teilchendurchmesser von 15 bis 20 µm, gemessen mittels Rasterelektronenmikroskopie, enthält.

14. Kosmetische Zubereitung oder Kombination nach einem der vorhergehenden Ansprüche, **dadurch gekennzeichnet, dass** die Zubereitung Hexyl 2-[4-(diethylamino)-2-hydroxybenzoyl]benzoate (INCI: Diethylamino hydroxybenzoyl hexyl benzoate) und/oder 4-(tert.-Butyl)-4'-methoxydibenzoylmethan enthält.

15. Kosmetische Zubereitung oder Kombination nach einem der vorhergehenden Ansprüche, **dadurch gekennzeichnet, dass** die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol, Ethylhexylglycerin und/oder 4-Hydroxyacetophenon enthält.

16. Kosmetische Zubereitung oder Kombination nach einem der vorhergehenden Ansprüche, **dadurch gekennzeichnet, dass** die Zubereitung Tapiokastärke und/oder Silica Dimethyl Silylate enthält.

17. Kosmetische Zubereitung oder Kombination nach einem der vorhergehenden Ansprüche, **dadurch gekennzeichnet, dass** die Zubereitung ein oder mehrere Verbindungen enthält, gewählt aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Ethylenbrassylat, Evernia Furfuracea Extract, Evernia Prunastri Extract, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

## Claims

1. Cosmetic sunscreen comprising
a) bismuth oxychloride particles having a refractive index of 2.15 and
b) titanium dioxide and/or zinc oxide having a primary particle size of 10 to 200 nm,
**characterized in that** the composition is free of polyethylene glycol, polyethylene glycol ethers and polyethylene glycol esters (called PEG derivatives) and does not contain any 3-(4-methylbenzylidene)camphor, 2-hydroxy-4-methoxybenzophenone (INCI: Oxybenzone), 2-ethylhexyl 4-methoxycinnamate, isoamyl 4-methoxycinnamate and ethylhexyl 2-cyano-3,3-diphenylacrylate (INCI: Octocrylene), any parabens, methylisothiazolinone, chlorphenesin, chloromethylisothiazolinone and DMDM hydantoin.

2. Combination of
a) bismuth oxychloride particles having a refractive index of 2.15 and
b) titanium dioxide and/or zinc oxide having a primary particle size of 10 to 200 nm in cosmetic preparations for use for the protection against radiation in the wavelength range of 401 to 500 nm.

3. Cosmetic preparation according to Claim 1 or combination according to Claim 2, **characterized in that** the bismuth oxychloride has a specific surface area of 230-350 m²/kg.

4. Cosmetic preparation or combination according to any of the preceding claims, **characterized in that** the bulk density of the bismuth oxychloride is 7.70-7.75 g/cm³.

5. Cosmetic preparation or combination according to any of the preceding claims, **characterized in that** the preparation the bismuth oxychloride is present in the preparation at a concentration of 0.1% to 10% by weight, based on the total weight of said preparation.

6. Cosmetic preparation or combination according to any of the preceding claims, **characterized in that** titanium dioxide and/or zinc oxide are/is present in the preparation in a total amount of 1% to 30% by weight, based on the total weight of said preparation.

7. Cosmetic preparation or combination according to any of the preceding claims, **characterized in that** the preparation additionally comprises interference pigments.

8. Cosmetic preparation or combination according to any of the preceding claims, **characterized in that** the preparation comprises 2,2'-methylenebis(6-(2H-benzotriazol-2-yl)-4-(1,1,3,3-tetramethylbutyl)-phenol) (INCI: Methylene Bis-Benzotriazolyl Tetramethylbutylphenol) and/or 2,4,6-tris(biphenyl)-1,3,5-triazine (INCI: Tris-Biphenyl Triazine).

9. Cosmetic preparation or combination according to any of the preceding claims, **characterized in that** the preparation comprises one or more UV filters selected from the following group of compounds: 2-phenylbenzimidazole-5-sulfonic acid and/or salts thereof; phenylene-1,4-bis(2-benzimidazyl)-3,3'-5,5'-tetrasulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)-sulfonic acid salts; 2-(2H-benzotriazol-2-yl)-4-methyl-6-[2-methyl-3-[1,3,3,3-tetramethyl-1-[(trimethylsilyl)oxy]disiloxanyl]propyl]phenol; 3-benzylidenecamphor; ethylhexyl salicylate; terephthalidenedicamphorsulfonic acid and/or salts thereof; 2-ethylhexyl 4-(dimethylamino)benzoate; 2-ethylhexyl 2-cyano-3,3-diphenylacrylate (Octocrylene), amyl 4-(dimethylamino)benzoate; di(2-ethylhexyl) 4-methoxybenzalmalonate; 2-hydroxy-4-methoxy-4'-methylbenzophenone; 2,2'-dihydroxy-4-methoxybenzophenone; homomenthyl salicylate (Homosalate); 2-ethylhexyl 2-hydroxybenzoate; dimethicodiethylbenzalmalonate; 3-(4-(2,2-bisethoxycarbonylvinyl)phenoxy)propenyl)methoxysiloxane / dimethylsiloxane copolymer; 4-(tert-butyl)-4'-methoxydibenzoylmethane; dioctylbutylamido-triazone (INCI: Diethylhexyl Butamido Triazone); 2,4-bis[5-1(dimethylpropyl)benzoxazol-2-yl-(4-phenyl)imino]-6-(2-ethylhexyl)imino-1,3,5-triazine with (CAS No. 288254-16-0); 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); merocyanines.

10. Cosmetic preparation or combination according to any of the preceding claims, **characterized in that** the preparation is in the form of an alcoholic solution or an emulsion.

11. Combination according to any of the preceding claims, **characterized in that** the preparation is free of polyethylene glycol, polyethylene glycol ethers and polyethylene glycol esters (called PEG derivatives).

12. Combination according to any of the preceding claims, **characterized in that** the preparation does not comprise any 3-(4-methylbenzylidene)camphor, 2-hydroxy-4-methoxybenzophenone (INCI: Oxybenzone), 2-ethylhexyl 4-methoxycinnamate, isoamyl 4-methoxycinnamate and ethylhexyl 2-cyano-3,3-diphenylacrylate (INCI: Octocrylene), any parabens, methylisothiazolinone, chlorphenesin, chloromethylisothiazolinone or DMDM hydantoin.

13. Cosmetic preparation or combination according to any of the preceding claims, **characterized in that** the preparation comprises spherical, hollow silica particles having an average particle diameter of 15 to 20 µm, measured by means of scanning electron microscopy.

14. Cosmetic preparation or combination according to any of the preceding claims, **characterized in that** the preparation comprises hexyl 2-[4-(diethylamino)-2-hydroxybenzoyl]benzoate (INCI: Diethylamino Hydroxybenzoyl Hexyl Benzoate) and/or 4-(tert-butyl)-4'-methoxydibenzoylmethane.

15. Cosmetic preparation or combination according to any of the preceding claims, **characterized in that** the preparation comprises one or more alkanediols from the following group of compounds: pentane-1,2-diol, hexane-1,2-diol, octane-1,2-diol, decane-1,2-diol, 2-methylpropane-1,3-diol, ethylhexylglycerin and/or 4-hydroxyacetophenone.

16. Cosmetic preparation or combination according to any of the preceding claims, **characterized in that** the preparation comprises tapioca starch and/or Silica Dimethyl Silyate.

17. Cosmetic preparation or combination according to any of the preceding claims, **characterized in that** the preparation comprises one or more compounds selected from the following group of compounds: limonene, citral, linalool, alpha-isomethylionone, geraniol, citronellol, 2-isobutyl-4-hydroxy-4-methyltetrahydropyran, 2-tert-pentylcyclohexyl acetate, 3-methyl-5-phenyl-1-pentanol, 7-acetyl-1,1,3,4,4,6-hexamethyltetralin, adipic acid diesters, alpha-amylcinnamaldehyde, alpha-methylionone, amyl C, butylphenyl methylpropional cinnamal, amyl salicylate, amylcinnamyl alcohol, anise alcohol, benzoin, benzyl alcohol, benzyl benzoate, benzyl cinnamate, benzyl salicylate, bergamot oil, bitter orange oil, butylphenyl methylpropional, cardamom oil, cedrol, cinnamal, cinnamyl alcohol, citronellyl methylcrotonate, lemon oil, coumarin, diethyl succinate, ethyl linalool, eugenol, ethylene brassylate, Evernia furfuracea extract, Evernia prunastri extract, farnesol, guaiac wood oil, hexyl cinnamal, hexyl salicylate, hydroxycitronellal, lavender oil, lime oil, linalyl acetate, mandarin oil, menthyl PCA, methylheptenone, nutmeg oil, rosemary oil, sweet orange oil, terpineol, tonka bean oil, triethyl citrate and/or vanillin.

## Revendications

1. Agent cosmétique de protection solaire contenant
a) des particules d'oxychlorure de bismuth ayant un indice de réfraction de 2,15, ainsi que
b) du dioxyde de titane et/ou l'oxyde de zinc ayant une granulométrie primaire de 10 à 200 nm,
**caractérisé en ce que** la préparation est exempte de polyéthylèneglycol, d'éthers de polyéthylèneglycol et d'esters de polyéthylèneglycol (ce que l'on appelle les dérivés PEG), et ne contient pas de 3-(4-méthylbenzylidène)-camphre, de 2-hydroxy-4-méthoxybenzophénone (INCI : Oxybenzon), de l'ester 2-éthylhexylique de l'acide 4-méthoxycinnamique, de l'ester isoamylique de l'acide 4-méthoxycinnamique et de 2-cyano-3,3-diphénylacrylate d'éthylhexyle (INCI : Octocrylen), de parabens, de méthylisothiazolinone, de chlorophénésine, de chlorométhylisothiazolinone et de DMDM-hydantoïne.

2. Combinaison de
a) particules d'oxychlorure de bismuth ayant un indice de réfraction de 2,15 et
b) du dioxyde de titane et/ou de l'oxyde de zinc ayant une granulométrie primaire de 10 à 200 nm dans des préparations cosmétiques destinées à une utilisation pour la protection vis-à-vis d'un rayonnement dans la gamme de longueurs d'onde de 401 à 500 nm.

3. Préparation cosmétique selon la revendication 1 ou combinaison selon la revendication 2, **caractérisée en ce que** l'oxychlorure de bismuth présente une aire spécifique de 230-350 m²/kg.

4. Préparation cosmétique ou combinaison selon l'une des revendications précédentes, **caractérisée en ce que** la masse volumique apparente de l'oxychlorure de bismuth est de 7,70-7,75 g/cm³.

5. Préparation cosmétique ou combinaison selon l'une des revendications précédentes, **caractérisée en ce que** la préparation l'oxychlorure de bismuth est contenu dans la préparation à une concentration de 0,1 à 10 % en poids par rapport au poids total de la préparation.

6. Préparation cosmétique ou combinaison selon l'une des revendications précédentes, **caractérisée en ce que** le dioxyde de titane et/ou l'oxyde de zinc sont contenus dans la préparation selon une quantité totale de 1 à 30 % en poids par rapport au poids total de la préparation.

7. Préparation cosmétique ou combinaison selon l'une des revendications précédentes, **caractérisée en ce que** la préparation contient en outre des pigments d'interférence.

8. Préparation cosmétique ou combinaison selon l'une des revendications précédentes, **caractérisée en ce que** la préparation contient du 2,2'-méthylène-bis-(6-(2H-benzotriazol-2-yl)-4-(1,1,3,3-tétraméthylbutyl)-phénol) (INCI : Methylene Bis-Benzotriazolyl Tetramethylbutylphenol) et/ou du 2,4,6-tris-(biphényle)-1,3,5-triazine (INCI : Tris-Biphenyl Triazine).

9. Préparation cosmétique ou combinaison selon l'une des revendications précédentes, **caractérisée en ce que** la préparation contient un ou plusieurs filtres UV qui sont choisis dans le groupe des composés acide 2-phénylbenzimidazole-5-sulfonique et/ou ses sels ; sels de l'acide phénylène-1,4-bis-(2-benzimidazyl)-3,3'-5,5'-tétrasulfonique ; 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-benzylidènecamphre ; salicylate d'éthylhexyle ; acide téréphtalidènedicamphosulfonique et/ou ses sels ; ester 2-éthylhexylique de l'acide 4-(diméthylamino)-benzoïque ; 2-cyano-3,3-diphénylacrylate de 2-éthylhexyle (octocrylène), ester amylique de l'acide 4-(diméthylamino)benzoïque ; ester di(2-éthylhexylique) de l'acide 4-méthoxybenzalmalonique ; 2-hydroxy-4-méthoxy-4'-méthylbenzophénone ; 2,2'-dihydroxy-4-méthoxybenzophénone ; salicylate d'homomenthyle (homosalate) ; 2-hydroxybenzoate de 2-éthylhexyle ; malonate de diméthicodiéthylbenzal ; copolymère 3-(4-(2,2-bis-éthoxycarbonylvinyl)-phénoxy)propényl)-méthoxysiloxane/diméthylsiloxane ; 4-(tert-butyl)-4'-méthoxydibenzoylméthane ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine portant le numéro (CAS 288254-16-0) ; 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin) ; ester tris(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone) ; mérocyanines.

10. Préparation cosmétique ou combinaison selon l'une des revendications précédentes, **caractérisée en ce que** la préparation se présente sous la forme d'une solution alcoolique ou d'une émulsion.

11. Combinaison selon l'une des revendications précédentes, **caractérisée en ce que** la préparation est exempte de polyéthylèneglycol, d'éthers de polyéthylèneglycol et d'esters de polyéthylèneglycol (ce que l'on appelle les dérivés PEG).

12. Combinaison selon l'une des revendications précédentes, **caractérisée en ce que** la préparation ne contient pas de 3-(4-méthylbenzylidène)-camphre, de 2-hydroxy-4-méthoxybenzophénone (INCI : Oxybenzon), de l'ester 2-éthylhexylique de l'acide 4-méthoxycinnamique, de l'ester isoamylique de l'acide 4-méthoxycinnamique et du 2-cyano-3,3-diphénylacrylate d'éthylhexyle (INCI : Octocrylen), pas de parabens, de méthylisothiazolinone, de chlorophénésine, de chlorométhylisothiazolinone et de DMDM-hydantoïne.

13. Préparation cosmétique ou combinaison selon l'une des revendications précédentes, **caractérisée en ce que** la préparation contient des particules creuses sphériques de silice ayant un diamètre moyen de particule de 15 à 20 µm, mesuré au microscope électronique à balayage.

14. Préparation cosmétique ou combinaison selon l'une des revendications précédentes, **caractérisée en ce que** la préparation contient du 2-[4-(diéthylamino)-2-hydroxybenzoyl]benzoate d'hexyle (INCI : Diethylamino hydroxybenzoyl hexyl benzoate) et/ou du 4-(tert-butyl)-4'-méthoxydibenzoylméthane.

15. Préparation cosmétique ou combinaison selon l'une des revendications précédentes, **caractérisée en ce que** la préparation contient un ou plusieurs alcanediols du groupe des composés 1,2-pentanediol, 1,2-hexanediol, 1,2-octanediol, 1,2-décanediol, 2-méthyl-1,3-propanediol, éthylhexylglycérol et/ou 4-hydroxyacétophénone.

16. Préparation cosmétique ou combinaison selon l'une des revendications précédentes, **caractérisée en ce que** la préparation contient de l'amidon de tapioca et/ou du diméthyl silylate de silice.

17. Préparation cosmétique ou combinaison selon l'une des revendications précédentes, **caractérisée en ce que** la préparation contient un ou plusieurs composés choisis dans le groupe des composés limonène, citral, linalool, alpha-isométhylionone, géraniol, citronellol, 2-isobutyl-4-hydroxy-4-méthyltétrahydropyrane, acétate de 2-tert-pentylcyclohexyle, 3-méthyl-5-phényl-1-pentanol, 7-acétyl-1,1,3,4,4,6-hexaméthyltétraline, diester de l'acide adipique, alpha-amylcinnamaldéhyde, alpha-méthylionone, amyl-C, butylphénylméthylpropionalcinnamal, salicylate d'amyle, alcool amylcinnamylique, alcool anisique, benzoïne, alcool benzylique, benzoate de benzyle, cinnamate de benzyle, salicylate de benzyle, essence de bergamote, essence d'orange amère, butylphénylméthylpropional, essence de cardamome, cédrol, cinnamal, alcool cinnamylique, méthylcrotonate de citronellyle, essence de citron, coumarine, succinate de diéthyle, éthyllinalool, eugénol, brassylate d'éthylène, extrait d'Evernia furfuracea, extrait d'Evernia prunastri, farnésol, huile de bois de gaïac, hexylcinnamal, salicylate d'hexyle, hydroxycitronellal, essence de lavande, essence de lémonène, acétate de linayle, essence de mandarine, menthyl PCA, méthylhepténone, essence de noix de muscade, essence de romarin, essence d'orange douce, terpinéol, essence de fève de Tonka, citrate de triéthyle et/ou vanilline.
